# FASCICULE DE BREVET EUROPEEN

(11) **EP 0 711 540 B1**
(45) Date de publication et mention de la délivrance du brevet: **10.05.2000**
(21) Numéro de dépôt: 95402521.9
(22) Date de dépôt: 10.11.1995
(51) Int. Cl.: A61K 7/00, A61K 9/127

(54) **Composition cosmétique ou dermatologique sous forme d'une dispersion d'une phase huileuse dans une phase aqueuse stabilisée à l'aide de particules de gel cubique et son procédé d'obtention**
Kosmetische oder dermatologische kubische Gel Partikeln-stabilisierte Öl/Wasser Dispersion und Verfahren zur Herstellung
Cosmetic or dermatologic oil/water dispersion stabilized with cubic gel particles and method of preparation

(30) Priorité: 10.11.1994 FR 9413564
(43) Date de publication de la demande: 15.05.1996
(73) Titulaire: L'OREAL, 75008 Paris (FR)
(72) Inventeur: Ribier, Alain, F-75001 Paris (FR); Biatry, Bruno, F-75012 Paris (FR)
(74) Mandataire: Stalla-Bourdillon, Bernard

(56) Documents cités:
- WO-A-84/02076
- LIPID TECHNOLOGY, vol. 2, no. 2, Avril 1990 pages 42-45, S. ENGSTRÖM 'Drug Delivery from Cubic and other Lipid-Water Phases.'

## Description

La présente invention a pour objet une composition se présentant sous forme d'une dispersion d'une phase huileuse dans une phase aqueuse, la phase huileuse étant stabilisée à l'aide de particules de gel cubique formées à l'aide d'un agent tensioactif hydrosoluble à chaîne grasse ainsi que son procédé de préparation.

Une grande variété de produits se présente sous forme d'une dispersion d'une phase huileuse dans une phase aqueuse telle que sous forme d'une émulsion. Il s'agit tout particulièrement de produits cosmétiques, dermatologiques ou pharmaceutiques, ces dispersions conférant à la peau de bonnes propriétés sensorielles et sont faciles d'application.

Toutefois, il est bien connu que les dispersions, et les émulsions en particulier, manquent de stabilité dans le temps du fait notamment des variations de température, celles-ci "cassent", donnant naissance à deux phases distinctes les rendant inutilisables.

La nature et la concentration de l'agent émulsionnant utilisé peut influer de façon significative sur la stabilité de telles compositions.

Toutefois, il est bien certain que le choix et la concentration d'un émulsionnant approprié va dépendre de divers facteurs et en particulier de l'huile ou des huiles constituant la phase huileuse de la dispersion ou de l'émulsion.

Par ailleurs, il doit être remarqué que certains tensioactifs ne sont pas sans inconvénients notamment lorsqu'ils sont employés à forte concentration en vue d'améliorer la stabilité.

En effet, sur les peaux sensibles, ils peuvent provoquer certains phénomènes d'irritation.

On a maintenant constaté de façon surprenante et inattendue qu'il était possible d'obtenir des dispersions d'une phase huileuse dans une phase aqueuse, particulièrement stables et non irritantes, à l'aide d'une très grande variété d'huiles en utilisant des particules de gel cubique contenant une faible proportion d'un agent tensioactif hydrosoluble à chaîne grasse. Les dispersions ainsi obtenues présentent en outre des qualités sensorielles particulièrement satisfaisantes.

Le terme de gel cubique utilisé selon la présente invention désigne des gels transparents, isotropes en lumière polarisée, se présentant sous forme de phase cristal liquide cubique. Les phases cubiques sont organisées d'une manière bi-polaire en domaines hydrophile et lipophile distincts, en contact étroit et formant un réseau tridimensionnel thermodynamiquement stable. Une telle organisation a été notamment décrite dans "La Recherche", Vol.23, pp.306-315, Mars 1992 et dans "Lipid Technology", Vol.2, n°2, pp.42-45, Avril 1990. Selon l'arrangement des domaines hydrophile et lipophile, la phase cubique est dite de type normal ou inverse. Le terme de gel cubique utilisé selon la présente invention regroupe bien entendu les gels présentant les différents types de phases cubiques.

L'utilisation de phases cubiques pour la réalisation de compositions, à délivrance retardée, à base de substances biologiquement actives a été décrite dans la demande WO 84/02076. Selon cette demande, les compositions sont à une seule phase, de préférence aqueuse et la substance biologiquement active est soit dissoute soit dispersée dans la phase cubique, cette dernière étant essentiellement obtenue à partir de monooléine, de lécithine de jaune d'oeuf, d'eau et de glycérol.

La présente invention se distingue de cet état de la technique non seulement par le fait que la composition est constituée de deux phases distinctes mais également par la nature des substances conduisant à la formation des particules de gel cubique.

La présente invention a donc pour objet une composition sous forme d'une dispersion comprenant :
(a) de 60 à 98 % en poids d'une phase aqueuse, et
(b) de 2 à 40 % en poids d'une phase huileuse,
ladite phase huileuse étant dispersée dans ladite phase aqueuse et stabilisée à l'aide de particules de gel cubique, lesdites particules étant essentiellement formées par :
(i) 0,1 à 15 % en poids par rapport au poids total de la composition, d'au moins un composé choisi parmi le 3,7,11,15-tétraméthyl-1,2,3-hexadecanetriol ou phytantriol, les dérivés N-2-alcoxycarbonyles de N-méthylglucamine et les monoglycérides d'acide gras insaturé, et
(ii) 0,05 à 3 % en poids par rapport au poids total de la composition d'un agent dispersant et stabilisant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

Selon un mode de réalisation particulier des compositions selon l'invention, la proportion pondérale relative en composé (i) par rapport au poids de la phase huileuse est comprise entre 0,02/1 et 1/1, et de préférence comprise entre 0,05/1 et 0,5/1.

Selon un mode de réalisation particulier des compositions selon l'invention, la proportion pondérale relative en composé (i) par rapport au poids dudit agent dispersant et stabilisant est comprise entre 2 et 200, et de préférence inférieure ou égale à 50.

Le phytantriol des particules de gel cubique est un composé connu, notamment commercialisé sous la dénomination de "Phytantriol-63926®" par la Société Roche.

Parmi les dérivés N-2-alcoxycarbonyles de N-méthylglucamine, on peut notamment citer ceux répondant à la formule (I) suivante : 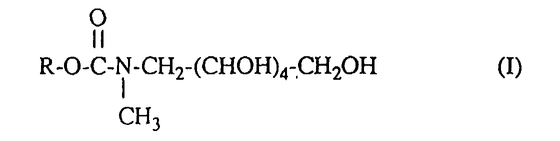 dans laquelle :
R représente un radical alkyle ramifié en C₆-C₁₈.

Parmi ceux-ci, on peut notamment mentionner la N-2-hexyldécyloxycarbonyl-N-méthyl-glucamine, la N-2-éthylhexyloxycarbonyl-N-méthyl-glucamine et la N-2-butyloctyloxycarbonyl-N-méthyl-glucamine.

Les composés de formule (I) tels que définis ci-dessus sont nouveaux et peuvent être préparés selon un procédé comprenant les étapes consistant :
(a) à dissoudre dans un mélange d'eau et d'un solvant organique, de la N-méthyl-glucamine,
(b) à disperser dans le mélange obtenu précédemment du bicarbonate de sodium, en une proportion appropriée correspondant environ à quatre fois la proportion molaire de N-méthyl-glucamine,
(c) à introduire alors dans le mélange réactionnel obtenu, un chloroformiate d'alkyle, le radical alkyle étant en C₆-C₁₈, en une proportion appropriée, généralement équimolaire par rapport à celle de N-méthyl-glucamine, puis à laisser réagir le mélange, et
(d) à récupérer le dérivé N-2-alcoxycarbonyle de N-méthylglucamine formé.

Le solvant organique utilisé dans l'étape (a) est généralement du tétrahydrofuranne.

L'étape (d) consiste à filtrer le mélange réactionnel obtenu après l'étape (c), à recueillir le résidu pâteux par filtration puis à le dissoudre dans de l'acétone en vue de sa cristallisation à une température de l'ordre de 5°C. Après filtration, les cristaux sont essorés et séchés sous vide. Des exemples de préparation de certains dérivés N-2-alcoxycarbonyles de N-méthyl-glucamine seront donnés ci-après dans la partie expérimentale.

Selon un mode de réalisation particulier des compositions selon l'invention, les particules de gel cubique contiennent comme composé (i), un mélange de phytantriol en une proportion de 1 à 40 % en poids par rapport au poids du mélange, et d'au moins un dérivé N-2-alcoxycarbonyle de N-méthyl-glucamine de formule (I) en une proportion de 60 à 99 % en poids par rapport au poids du mélange.

Selon une forme préférée de ce mode de réalisation, la proportion en phytantriol est de 10 à 30 % en poids par rapport au poids du mélange et celle du dérivé N-2-alcoxycarbonyle de N-méthyl-glucamine de 70 à 90 % en poids par rapport au poids du mélange.

Les monoglycérides d'acide gras insaturé sont de préférence ceux ayant une chaîne grasse insaturée en C₁₆-C₂₂.

Parmi ceux-ci, on peut notamment citer le monooléate de glycéryle ou monooléine et le monolinoléate de glycéryle ou monolinoléine.

On peut bien entendu utiliser dans les compositions selon l'invention, un mélange de monoglycérides tels que définis précédemment ainsi qu'un mélange de monoglycérides d'acide gras insaturé et de monoglycérides d'acide gras saturé, la proportion en monoglycérides d'acide gras saturé étant cependant de préférence inférieure à celle de monoglycérides d'acide gras insaturé.

Selon un autre mode de réalisation des compositions selon l'invention, les particules de gel cubique contiennent comme composé (i), un mélange de phytantriol en une proportion de 1 à 50 % en poids par rapport au poids total du mélange et d'au moins un monoglycéride d'acide gras insaturé en une proportion de 50 à 99% en poids par rapport au poids du mélange.

Selon une forme préférée de ce mode de réalisation, la proportion en phytantriol est de 10 à 30 % en poids par rapport au poids du mélange et celle en monoglycéride d'acide gras insaturé de 70 à 90 % en poids par rapport au poids du mélange.

L'agent dispersant et stabilisant (ii) est de préférence choisi parmi :
(1) les alkyl ou alcényl éthers ou esters de polyol,
(2) les amino-acides N-acylés et leurs dérivés et les peptides N-acylés par un radical alkyle ou alcényle, et leurs sels,
(3) les alkyl ou alcényl éthers ou esters sulfates, leurs dérivés et leurs sels,
(4) les alkyl ou alcényl éthers ou esters gras polyoxyéthylénés,
(5) les acides alkyl ou alcényl carboxyliques polyoxyéthylénés et leurs sels,
(6) les N-alkyl ou alcényl bétaïnes,
(7) les alkyl ou alcényl triméthylammonium et leurs sels, et
(8) leurs mélanges.

Dans les composés ci-dessus énumérés, les radicaux alkyle et alcényle ont de 8 à 22 atomes de carbone et peuvent se présenter sous forme de mélanges.

### 1- Alkyl ou alcényl éthers ou esters de polyol

Parmi ceux-ci, on peut citer notamment :
(a) les alkyl ou alcényl esters de sorbitan polyoxyéthylénés par au moins 20 motifs d'oxyde d'éthylène tels que le palmitate de sorbitan 20 OE ou Polysorbate 40 commercialisé sous la dénomination de "Montanox 40 DF®" par la Société Seppic, et le laurate de sorbitan 20 OE ou Polysorbate 20 commercialisé sous la dénomination de "Tween 20®" par la Société ICI,
(b) les alkyl ou alcényl esters de polyglycérol comportant au moins 10 motifs dérivés du glycérol, oxyéthylénés ou non, tels que le polyglycéryl-10 laurate commercialisé sous la dénomination de "Decaglyn 1-L®" par la Société Nikko Chemicals,
(c) les alkyl ou alcényl éthers de polyglycérol tels que le polyglycéryl-3 hydroxylauryléther commercialisé sous la dénomination de "Chimexane NF®" par la Société Chimex, et
(d) les alkyl ou alcényl éthers ou esters de mono ou polysaccharides tels que ceux dérivant du glucose, du fructose, du galactose, du maltose ou du lactose et notamment les monoesters en 1- et 6- du D-fructose, du décylglucose et du décylpolyglucose.

### 2- Amino-acides N-acylés et leurs dérivés et les peptides N-acylés par un radical alkyle ou alcényle et leurs sels

Parmi ceux-ci, on utilise de préférence ceux pour lesquels le radical alkyle ou alcényle a au moins 12 atomes de carbone.

Par amino-acides, on entend selon l'invention les α, β ou γ-amino-acides. Comme sels d'amino-acides N-acylés, on peut citer par exemple ceux de glutamate N-acylé tels que le cocoylglutamate de monosodium, le lauroylglutamate de monosodium, l'alcoyl C₁₄-C₂₀ glutamate de disodium (le radical alcoyle C₁₄-C₂₀ dérivant du suif hydrogéné), commercialisés respectivement sous les dénominations de "Acylglutamate CS-11®", de "Acylglutamate LS-11®" et de "Acylglutamate HS-21®" par la Société Ajinomoto.

On peut encore citer les lysines N-acylées telles que la lauroyl lysine commercialisée sous la dénomination de "Amihope LL®" par la Société Ajinomoto.

Les dérivés d'amino-acides N-acylés et leurs sels sont de préférence les sarcosinates N-acylés tels que le lauroyl sarcosinate de sodium commercialisé sous la dénomination de "Oramix L30®" par la Société Seppic, le myristoyl sarcosinate de sodium et le palmitoyl sarcosinate de sodium commercialisés respectivement sous les dénominations de "Nikkol Sarcosinate MN®" et de "Nikkol Sarcosinate PN®", par la Société Nikko Chemicals.

Parmi les peptides N-acylés, on peut citer ceux dérivés de tout ou partie du collagène ou de la kératine tels que le lauroyl collagène de sodium et la palmitoyl kératine commercialisés sous les dénominations de "Proteol B 30®" et de "Lipacide PK®" par la Société Seppic.

### 3- Alkyl ou alcényl éthers ou esters sulfates, leurs dérivés et leurs sels

Parmi ceux-ci, on utilise de préférence ceux pour lesquels le radical alkyle ou alcényle a au moins 12 atomes de carbone.

Parmi les alkyl ou alcényl éthers sulfates, on utilise de préférence les sels d'alkyl éther sulfate et notamment le lauryléther sulfate de sodium.

Parmi les alkyl ou alcényl esters sulfates, on peut citer par exemple les esters de l'acide iséthionique ainsi que ses sels et notamment le cocoyl iséthionate de sodium commercialisé sous la dénomination de "Geropon AC 78®" par la Société Rhône Poulenc.

### 4- Alkyl ou alcényl éthers ou esters gras polyoxyéthylénés

Parmi ceux-ci, on utilise de préférence ceux pour lesquels le radical alkyle ou alcényle a au moins 12 atomes de carbone. Ceux particulièrement préférés ont au moins 20 motifs d'oxyde d'éthylène tels que par exemple le PEG-20 stéarate, le laureth-23, l'oleth-20 et le PEG-25 phytostérol.

### 5- Acides alkyl ou alcényl carboxyliques polyoxyéthylénés et leurs sels

Parmi ceux-ci, on utilise de préférence ceux comportant au moins 10 motifs d'oxyde d'éthylène tels que par exemple l'acide laureth-10 carboxylique et l'acide oleth-10 carboxylique.

### 6- N-alkyl ou alcényl bétaïnes

Parmi celles-ci, on utilise de préférence celles pour lesquelles le radical alkyle ou alcényle a au moins 12 atomes de carbone telles que par exemple la lauryl amidopropyl bétaïne et l'oléyl amidopropyl bétaïne.

### 7- Alkyl ou alcényl triméthylammonium et leurs sels

Parmi ceux-ci, on utilise de préférence ceux pour lesquels le radical alkyle ou alcényle a au moins 12 atomes de carbone. Comme sels on utilise de préférence les bromures et chlorures tels que le chlorure de cocoyltriméthylammonium et le bromure de cétyltriméthylammonium.

Lorsque le composé (i) est un dérivé N-2-alcoxycarbonyle de N-méthyl glucamine de formule (I), on utilise de préférence comme agent dispersant et stabilisant (ii), le polyglycéryl-3 hydroxylauryléther, le lauryléthersulfate de sodium ou le bromure de cétyltriméthylammonium.

Selon un mode de réalisation particulier des compositions selon l'invention, les particules de gel cubique comprennent en outre de 0,0005 % à 5 % en poids et de préférence de 0,001 % à 2 % en poids d'un lipide amphiphile ionique non hydrosoluble.

Parmi ceux-ci, on peut notamment citer :
(i) les phospholipides tels que les phospholipides naturels comme la lécithine de soja ou d'oeuf, les phospholipides modifiés par voie chimique ou enzymatique comme la lécithine hydrogénée ou le sel de sodium de l'acide phosphatidique, et les phospholipides de synthèse comme la dipalmitoylphosphatidylcholine,
(ii) les esters phosphoniques d'acide gras tels que le monocétyl phosphate et ses sels de sodium et de potassium commercialisé sous la dénomination de "Monafax 160®" par la Société Mona, ainsi que le dimyristyl phosphate et ses sels de sodium et de potassium commercialisé sous la dénomination de "Mexoryl SY®" par la Société Chimex,
(iii) les dérivés N-acylés de l'acide glutamique tels que le stéaroylglutamate de monosodium commercialisé sous la dénomination de "Acylglutamate HS 11®" par la Société Ajinomoto et le mélange cocoyl-alcoyl C₁₄-C₂₀ glutamate de monosodium, le radical alcoyle C₁₄-C₂₀ dérivant du suif hydrogéné, commercialisé sous la dénomination de "Acylglutamate GS 11®" par la Société Ajinomoto,
(iv) le cétylsulfate de sodium commercialisé sous la dénomination de "Nikkol SCS®" par la Société Nikko Chemicals,
(v) le cocoyl monoglycéride sulfate de sodium commercialisé sous la dénomination de "Nikkol SGC 80N®" par la Société Nikko Chemicals, et
(vi) les dérivés d'ammonium quaternaire tels que le chlorure de béhényltriméthylammonium, le chlorure de dilauryldiméthylammonium, le chlorure de distéaryldiméthylammonium, le méthylsulfate de 4,5-dihydro 1-méthyl 2-alcoyl C₁₄-C₂₀ 1-(2-alcoyl C₁₄-C₂₀ aminoéthyl)imidazolium, les radicaux alcoyles C₁₄-C₂₀ dérivant du suif hydrogéné, commercialisé sous la dénomination de "Rewoquat W75H®" par la Société Rewo Chemische, le méthylsulfate de dialkylhydroxyéthylméthylammonium dont les radicaux alkyles dérivent du suif, hydrogéné ou non, commercialisé sous la dénomination de "Stepanquat VP 85®" par la Société Stepan et le "Quaternium-82" commercialisé par la Société Seppic sous la dénomination de "Amonyl DM®".

L'incorporation de ces lipides amphiphiles ioniques non hydrosolubles confère aux particules de gel cubique une charge superficielle entraînant une répulsion électrostatique des particules entre-elles.

Les particules de gel cubique telles que définies précédemment ont généralement une taille moyenne, mesurée à l'aide d'un granulomètre laser BI 90 de la Société Brookhaven Instruments Corporation, d'environ 0,05 µm à environ 1 µm, et de préférence inférieure ou égale à 0,5 µm.

Il est en outre possible d'incorporer dans les particules de gel cubique, divers types de composés actifs. En particulier, lesdites particules peuvent contenir un principe actif hydrophile ou lipophile.

Bien entendu, grâce à la structure particulière des particules de gel cubique, il est possible d'incorporer dans celles-ci à la fois des principes actifs hydrophiles et lipophiles, même si ceux-ci présentent une certaine incompatibilité.

Parmi les différents principes actifs pouvant être incorporés, on peut notamment citer :
1) les agents antioxydants ou anti-radicaux libres tels que les protéines et les enzymes par exemple la superoxyde dismutase (SOD), la lactoperoxydase et la lactoferrine ; les peptides et leurs dérivés tels que la taurine et la carnosine ; les séquestrants tels que l'acide phytique et les dérivés polyphosphoniques ; les flavonoïdes tels que la rutine et l'α-glycosyl rutine ; la chlorophylline ; l'éthoxyquine ; la guanosine ; les tocophérols, notamment les α,β ou γ tocophérols et en particulier le d-α-tocophérol commercialisé sous la dénomination de "Cophérol 1300®" par la Société Henkel ainsi que l'acétate de tocophérol ; le di t-butyl hydroxybenzylidène camphre commercialisé sous la dénomination de "Mexoryl SAD®" par la Société Chimex et la t-butyl hydroquinone commercialisée sous la dénomination d'"Embanox®" par la Société Rhône-Poulenc ; le palmitate d'ascorbyle et le β-carotène,
2) les agents hydratants ou humectants tels que l'acide hyaluronique et son sel de sodium ; le β-glycérophosphate ; le glycérol et le sorbitol,
3) les filtres UV tels que les produits commercialisés sous les dénominations de "Eusolex 232®" par la Société Merck, de "Parsol 1789®" et de "Parsol MCX®" par la Société Givaudan-Roure, de "Mexoryl SX®" par la Société Chimex et de "Uvinul T150®" par la Société BASF,
4) les kératolytiques tels que les enzymes protéolytiques et notamment la subtilisine, la trypsine, l'α-chymotrypsine et la papaïne ; l'acide rétinoïque et les α-hydroxyacides notamment arômatiques tels que l'acide salicylique et ses dérivés en particulier l'acide n-dodécanoyl-5 salicylique,
5) les accélérateurs de bronzage tels que la caféine et les dérivés de tyrosine tels que le tyrosinate de glucose et le sel disodique de la N-L-malyl tyrosine,
6) les dépigmentants tels que l'acide kojique ; l'acide glycolique ; la vitamine C et notamment l'ascorbyle phosphate de magnésium, et l'arbutine et ses dérivés,
7) les colorants naturels tels que les matières colorantes extraites de végétaux comme la chlorophylline et le β-carotène ou extraites d'animaux comme le carmin de cochenille et le caramel,
8) les auto-bronzants tels que la dihydroxyacétone et les indoles,
9) les liporégulateurs tels que le γ-orizanol ; l'extrait de *Centella asiatica* contenant de la génine et de l'acide asiatique ; la caféine et la théophylline,
10) les agents anti-vieillissement et anti-rides tels que les hydroxyacides et notamment les α-hydroxyacides comme l'acide glycolique, l'acide salicylique et ses dérivés tels que l'acide n-octanoyl salicylique commercialisé sous la dénomination de "Mexoryl SAB®" par la Société Chimex, l'acide lactique et ses dérivés tels que le lactate stéarate de glycéryle et le lactate palmitate de glycéryle commercialisés par la Société Grinsted sous les dénominations respectives de "Lactodan B 30®" et "Lactodan F 15®" et le lactate d'octyldodécyle commercialisé sous la dénomination de "Cosmol 13®" par la Société Nisshin Oil Mills ; le rétinol et ses dérivés comme l'acétate, le palmitate et le propionate de rétinol, et les rétinoïdes,
11) les agents anti-inflammatoires et cicatrisants tels que l'acide 18 β-glycyrrhétinique et ses sels notamment son sel d'ammonium ; l'α-bisabolol ; les corticoïdes et l'extrait de *Centella asiatica,*
12) les antibactériens et antifongiques tels que le chlorure de benzalkonium, la chlorhexidine, l'hexetidine et l'hexamidine,
13) les insectifuges tels que les diéthyl et diméthyltoluamides,
14) les déodorants tels que l'hexachlorophène et le triclosan produit commercialisé sous la dénomination de "Irgasan DP 300®" par la Société Ciba-Geigy,
15) les antipelliculaires tels que l'octopirox et les dérivés de pyridinethione tels que ceux commercialisés sous les dénominations de "Omadine®" par la Société Olin,
16) les agents anti-chute des cheveux tels que le nicotinate de méthyle ou d'hexyle et le minoxidil,
17) les colorants capillaires tels que les bases et les coupleurs d'oxydation ; les colorants directs et les colorants auto-oxydables,
18) les agents réducteurs pour permanentes tels que l'acide thioglycolique ; la cystéine ; la cystéamine ; la N-acétyl cystéine ; la N-acétyl cystéamine et le thioglycolate de glycérol,
19) les agents conditionneurs pour peau et cheveux tels que les polymères cationiques et les cations, et
20) les huiles essentielles telles que l'huile de bergamote.

On peut utiliser dans les compositions selon l'invention soit des particules de gel cubique ne contenant pas de principes actifs, soit des particules contenant des principes actifs hydrophiles ou lipophiles, soit encore des particules contenant à la fois des principes actifs hydrophiles et lipophiles.

Dans les compositions selon l'invention, la phase huileuse se trouve être dispersée dans la phase aqueuse et se présente généralement sous forme de gouttelettes de taille moyenne comprise entre 0,1 µm et 10 µm.

La phase huileuse des compositions selon l'invention est essentiellement constituée d'au moins une huile d'origine végétale, animale, minérale ou de synthèse, de préférence cosmétiquement, dermatologiquement ou pharmaceutiquement acceptable.

Parmi les huiles végétales, on peut notamment citer l'huile de tournesol, l'huile de maïs, l'huile de soja, l'huile de courge, l'huile de pépins de raisin ou de cassis, l'huile de jojoba, l'huile d'amande douce, l'huile de carthame, l'huile de césame, l'huile de bourrache, l'huile de noisette, l'huile de macadamia et la fraction liquide du beurre de karité.

Comme huiles végétales, on peut également utiliser des huiles essentielles telles que l'huile d'eucalyptus, l'huile de lavandin, l'huile de lavande, l'huile de vétiver, l'huile de litsea cubeba, l'huile de citron, l'huile de santal, l'huile de romarin, l'huile de camomille, l'huile de sariette, l'huile de noix de muscade, l'huile de cannelle, l'huile d'hysope, l'huile de carvi, l'huile d'orange, l'huile de géraniol, l'huile de cade et l'huile de bergamote.

Parmi les huiles animales, on peut notamment citer les huiles de poisson, l'huile de tortue, l'huile de vison ainsi que le squalène hydrogéné (ou perhydrosqualène).

Comme huiles minérales, on peut notamment citer l'huile de paraffine et les isoparaffines.

Parmi les huiles de synthèse, on peut notamment citer des hydrocarbures tels que l'isohexadécane, le polydécène et le polyisobutène, des alcools gras tels que l'octyldodécanol, l'alcool isostéarylique et l'alcool oléique, des esters tels que les glycérides d'Acides Gras Essentiels, les triglycérides des acides caprique et caprylique et leurs mélanges, et des esters d'alcool gras et d'acide gras linéaire ou ramifié comme l'huile de purcellin (octanoate de stéaryle).

Comme huiles de synthèse, on peut également utiliser dans les compositions selon l'invention des huiles de silicone de type linéaire telles que le polydiméthylsiloxane, de type cyclique telles que le cyclopentadiméthyl siloxane et de type organomodifié telles que le polyphényltriméthyl siloxane et le polydiméthyl siloxane oxyéthyléné oxypropyléné.

On peut encore citer des huiles fluorées telles que les perfluorodécahydronaphtalènes comme la perfluorodécaline, ainsi que des huiles de type polymérique telles que les perfluoropolyméthylisopropyléthers.

Selon un mode de réalisation des compositions selon l'invention, il est également possible d'incorporer au moins un principe actif dans la phase huileuse et/ou dans la phase aqueuse. Ce principe actif peut être notamment choisi parmi les principes actifs tels que définis précédemment.

La phase aqueuse de la composition selon l'invention peut en outre contenir différents additifs conventionnels. Parmi ceux-ci, on peut notamment citer des agents conservateurs, des parfums, des pigments (TiO₂), des matières colorantes, des charges et des agents gélifiants.

Parmi les agents gélifiants pouvant être utilisés dans les compositions selon l'invention, on peut citer en particulier les dérivés de cellulose tels que l'hydroxyéthylcellulose et les alkylhydroxyéthylcelluloses, les dérivés d'algues tels que le satiagum, des gommes naturelles telles que l'adragante, des polymères synthétiques tels que les mélanges d'acides polycarboxyvinyliques et en particulier ceux commercialisés sous les dénominations de "Carbopol®" par la Société Goodrich ou de "Synthalen®" par la Société 3V SA.

La proportion en agent gélifiant est généralement comprise entre 0,1 et 2 % en poids par rapport au poids total de la composition.

La présente invention a également pour objet un procédé de préparation d'une composition sous forme de dispersion, celui-ci consistant en au moins deux étapes.

La première étape consiste à préparer une dispersion aqueuse de particules de gel cubique telles que définies précédemment, par fragmentation, à l'aide d'un homogénéiseur, d'un gel cubique formé à l'aide d'au moins un composé (i) tel que défini précédemment, d'eau, en présence éventuellement de lipides amphiphiles ioniques non hydrosolubles et/ou de principes actifs hydrophiles et/ou lipophiles et d'au moins un agent dispersant et stabilisant (ii) tel que défini précédemment. L'homogénéiseur peut être du type rotor-stator à fort gradient de cisaillement comme le "Virtis®" ou le "Heidolph Diax 600®" ou un homogénéiseur haute pression fonctionnant entre 200 et 1.800 bars environ (20 à 180 MPa).

La taille des particules de gel cubique peut être modulée par la nature et la concentration de l'agent dispersant et stabilisant (ii) utilisé.

Il est bien entendu possible d'introduire, à ce stade de la préparation de la dispersion aqueuse des particules de gel cubique, divers additifs et/ou principes actifs dans la phase aqueuse.

Après la formation des particules de gel cubique, l'agent dispersant et stabilisant se trouve, généralement, à l'extérieur desdites particules.

La deuxième étape consiste ensuite à ajouter à ladite dispersion obtenue, une phase huileuse contenant éventuellement certains additifs et/ou principes actifs lipophiles et à soumettre le mélange à une agitation mécanique qui peut être notamment réalisée à l'aide d'un homogénéiseur du même type que ceux définis ci-dessus.

Divers additifs et/ou principes actifs peuvent être également introduits à ce stade de la préparation.

En particulier, lorsque l'on souhaite préparer une dispersion gélifiée, on ajoute généralement au mélange obtenu après la seconde étape, une solution aqueuse contenant un agent gélifiant.

Les compositions selon l'invention sous forme de dispersion sont plus particulièrement destinées à une utilisation cosmétique, dermatologique ou pharmaceutique et se présentent sous différentes formes telles que notamment un lait, une crème ou un sérum.

On va maintenant donner à titre d'illustration des exemples de préparation de dérivés N-2-alcoxycarbonyles de N-méthyl-glucamine ainsi que plusieurs exemples de compositions sous forme de dispersion selon l'invention.

### EXEMPLES

### Préparation des dérivés N-2-alcoxycarbonyles de N-méthyl-glucamine.

### EXEMPLE A : Préparation de la N-2-hexyldécyloxycarbonyl-N-méthyl-glucamine

Dans un réacteur, on dissout 70,2 g de N-méthyl-glucamine (0,36 moles) dans un mélange constitué de 60 ml d'eau et de 80 ml de tétrahydrofuranne, puis on disperse 120,96 g de bicarbonate de sodium (1,44 moles).

Tout en maintenant la température du mélange réactionnel à 8°C, on ajoute goutte-à-goutte, 109,62 g de chloroformiate de 2-hexyldécanoyle (0,36 moles) et laisse réagir pendant 3 heures sous agitation à 5°C. Après avoir laissé reposer le mélange pendant une nuit à température ambiante, celui-ci est filtré et concentré. Le résidu pâteux est ensuite dissous dans 1 litre d'acétone. Après cristallisation par refroidissement, on filtre puis cristallise à nouveau dans 0,5 litre d'acétone. Le produit cristallisé est alors filtré puis séché.

On obtient ainsi 100 g de N-2-hexyldécyloxycarbonyl-N-méthyl-glucamine (Rendement : 60 %) de point de fusion : 70,6°C.

Selon le même mode opératoire que décrit ci-dessus, on a également préparé la N-2-éthylhexyloxycarbonyl-N-méthyl-glucamine (point de fusion : 74,2°C) et la N-2-butyloctyloxycarbonyl-N-méthyl-glucamine (point de fusion : 77°C).

### EXEMPLES DE COMPOSITIONS

### EXEMPLE 1:

Une dispersion aqueuse de particules de gel cubique est obtenue par mélange de 3 g de phytantriol et de 1,28 g d'eau, auquel on ajoute 75,7 g d'une solution aqueuse contenant 0,95 g de Polysorbate 40 commercialisé sous la dénomination de "Montanox 40 DF®" par la Société Seppic. Le mélange est alors prédispersé puis homogénéisé, à température ambiante, à l'aide d'un homogénéiseur de type "Virtis®" à 35.000 tr/mn pendant 5 minutes, cette agitation étant répétée 4 fois.

A la dispersion aqueuse de particules de gel cubique obtenue, on ajoute alors 0,02 g de conservateurs puis une phase huileuse constituée de 10 g d'huile d'amande d'abricot et de 10 g d'huile de silicone volatile commercialisée sous la dénomination de "Dow Corning Fluid 345®" par la Société Dow Corning. Après agitation à température ambiante à l'aide d'un homogénéiseur de type "Virtis®" à 35.000 tr/min pendant 5 minutes, cette agitation étant répétée 5 fois, on obtient une dispersion stable de bonne consistance et agréable à l'application.

La taille moyenne des gouttelettes de la phase huileuse est d'environ 0,51 µm (facteur de polydispersité : 0,6).

### EXEMPLE 2 :

Une dispersion aqueuse de particules de gel cubique est obtenue par mélange de 2,97 g de phytantriol et de 0,03 g de stéaroylglutamate de monosodium commercialisé sous la dénomination de "Acylglutamate HS-11®" par la Société Ajinomoto et de 1,28 g d'eau, auquel on ajoute 75,7 g d'une solution aqueuse contenant 0,95 g de Polysorbate 40. Le mélange est alors prédispersé puis homogénéisé, à température ambiante, à l'aide d'un homogénéiseur de type "Virtis®" à 35.000 tr/mn pendant 5 minutes, cette agitation étant répétée 4 fois.

A la dispersion aqueuse de particules de gel cubique obtenue, on ajoute alors 0,02 g de conservateurs puis une phase huileuse constituée de 10 g d'huile d'amande d'abricot et de 10 g d'huile de silicone volatile commercialisée sous la dénomination de "Dow Corning Fluid 345®" par la Société Dow Corning. Après agitation à température ambiante à l'aide d'un homogénéiseur de type "Virtis®" à 35.000 tr/min pendant 5 minutes, cette agitation étant répétée 5 fois, on obtient une dispersion stable et homogène.

La taille moyenne des gouttelettes de la phase huileuse est d'environ 0,37 µm (facteur de polydispersité : 0,05).

### EXEMPLES 3, 4, 5 et 6 (Comparatifs)

En prenant comme référence la composition de l'exemple 1 et selon le même mode opératoire, on a réalisé les compositions suivantes :

### Exemple 3 :

- Polysorbate 40 0,95 g
- Conservateurs 0,02 g
- Huile d'amande d'abricot 10 g
- Huile de silicone volatile commercialisée sous la dénomination de "Dow Corning Fluid 345®" par la Société Dow Corning 10 g
- Eau q.s.p. 100 g

### Exemple 4 :

- Polysorbate 40 0,95 g
- Stéaroylglutamate de monosodium 0,03 g
- Conservateurs 0,02 g
- Huile d'amande d'abricot 10 g
- Huile de silicone volatile commercialisée sous la dénomination de "Dow Corning Fluid 345®" par la Société Dow Corning 10 g
- Eau q.s.p. 100 g

### Exemple 5 :

- Stéaroylglutamate de monosodium 0,03 g
- Conservateurs 0,02 g
- Huile d'amande d'abricot 10 g
- Huile de silicone volatile commercialisée sous la dénomination de "Dow Corning Fluid 345®" par la Société Dow Corning 10 g
- Eau q.s.p. 100 g

On a ensuite calculé la taille moyenne des gouttelettes lipidiques dans les dispersions obtenues par mesure à l'aide d'un granulomètre laser BI 90 de la Société Brookhaven Instruments Corporation.

On a également évalué la stabilité des dispersions obtenues par observation macroscopique après 1 mois à température ambiante. On considère qu'une composition est stable lorsque l'on n'observe aucune séparation des phases aqueuse et huileuse après 1 mois au repos.

On a obtenu les résultats suivants :

| Exemples | Taille des gouttelettes lipidiques | Stabilité après 1 mois |
|---|---|---|
| Exemple 1 | 0,51µm | Stable |
| Exemple 3 | > 1µm | Instable |
| Exemple 4 | > 1µm | Instable |
| Exemple 5 | > 2µm | Instable |

La dispersion de l'exemple 3, qui est instable, diffère uniquement de celle de l'exemple 1 par l'absence de particules de gel cubique. Ceci montre l'importance de ces dernières pour la bonne stabilité de la dispersion.

La dispersion de l'exemple 4 est identique à celle de l'exemple 3 mais contient un lipide amphiphile ionique non hydrosoluble, à savoir le stéaroylglutamate de monosodium. La présence de ce dernier n'a aucune incidence sur la stabilité et cet exemple montre à nouveau l'importance des particules de gel cubique sur la stabilité.

La dispersion de l'exemple 5 est identique à celle de l'exemple 4 mais ne contient pas d'agent dispersant et stabilisant, à savoir le Polysorbate 40. Ici encore, la seule présence d'un lipide amphiphile ionique non hydrosoluble ne permet pas l'obtention d'une dispersion stable.

Il résulte de cette étude comparative que seule la présence de particules de gel cubique conduit à des dispersions de bonne stabilité.

### EXEMPLE 6 :Crème de jour

Une dispersion aqueuse de particules de gel cubique est obtenue par mélange à température ambiante de 2,97 g de phytantriol, de 0,03 g de stéaroylglutamate de monosodium commercialisé sous la dénomination de "Acylglutamate HS-11®" par la Société Ajinomoto, de 0,1 g d'acétate de tocophérol et de 1,3 g d'eau déminéralisée, auquel on ajoute à température ambiante 51 g d'une solution aqueuse contenant 3 g de glycérine, 0,01 g de guanosine et 1 g de polysorbate 40.

Le mélange est ensuite dispersé et homogénéisé à température ambiante à l'aide d'un homogénéiseur de type "Heidolph Diax 600®" muni d'une tête de dispersion 18 G, à 25.000 tr/min pendant 15 minutes, puis par 4 passages à 600 bars dans un homogénéiseur haute pression de type "Soavi®".

A la dispersion aqueuse de particules de gel cubique obtenue (dite dispersion A), on ajoute une solution B obtenue par mélange des ingrédients suivants :

### Solution B :

- Huile d'amande d'abricot 12 g
- Filtre solaire 1 g
- Huile de silicone volatile commercialisée sous la dénomination de "Dow Corning Fluid 345®" par la Société Dow Corning 12 g
- Parfum 0,3 g

Le mélange est alors homogénéisé à température ambiante à l'aide d'un homogénéiseur haute pression de type "Soavi®" par 4 passages à 600 bars.

On y ajoute ensuite une solution C obtenue par mélange des ingrédients suivants :

### Solution C :

- Cétylhydroxyéthylcellulose commercialisée sous la dénomination de "Natrosol Plus Grade 330 CS®" par la Société Aqualon 1 g
- Conservateurs 0,3 g
- Eau déminéralisée 18 g

On homogénéise alors le mélange à température ambiante à l'aide d'un agitateur à palette de type "Heidolph RZR 50®" à 50 tr/min pendant 30 minutes.

La dispersion obtenue sous forme d'une crème est stable et homogène. Elle s'applique facilement sur la peau, ne colle pas, ne poisse pas et protège la peau contre les effets néfastes des radicaux libres.

### EXEMPLE 7 : Crème de jour anti-vieillissement

Selon le même mode opératoire que décrit à l'exemple 6, on a préparé une crème de jour sous forme d'une dispersion par mélange des parties suivantes :

### Dispersion A

- Phytantriol 2,97 g
- Stéaroylglutamate de monosodium commercialisé sous la dénomination de "Acylglutamate HS-11®" par la Société Ajinomoto 0,03 g
- Vitamine E 0,1 g
- Glycérine 3 g
- Polysorbate 40 commercialisé sous la dénomination de "Montanox 40 DF®" par la Société Seppic 1 g
- Polyphosphonate commercialisé sous la dénomination de "Dequest 2046®" par la Société Monsanto 0,1 g
- Superoxyde dismutase commercialisée sous la dénomination de "CU-ZN SOD®" par la Société Bio-Technology 0,0005 g
- Eau déminéralisée 44,4995 g

### Solution B

- Huile de pépins de cassis 10 g
- Huile de jojoba 7 g
- Vitamine E 1 g
- Huile de silicone volatile commercialisée sous la dénomination de "Dow Corning Fluid 345®" par la Société Dow Corning 4 g
- Filtre solaire 1 g
- Parfum 0,3 g

### Solution C

- Cétylhydroxyéthylcellulose commercialisée sous la dénomination de "Natrosol Plus Grade 330 CS®" par la Société Aqualon 1 g
- Conservateur 0,3 g
- Eau déminéralisée 23,7 g

### EXEMPLE 8 : Lait hydratant

Selon le même mode opératoire que décrit à l'exemple 6, on a préparé un lait hydratant sous forme d'une dispersion par mélange des parties suivantes :

### Dispersion A

- Phytantriol 1,96 g
- Cétylphosphate commercialisé sous la dénomination de "Monafax 160®" par la Société Mona 0,04 g
- Céramide de synthèse commercialisée sous la dénomination de "Mexanyl GZ®" par la Société Chimex 0,2 g
- Glycérine 2 g
- L-hydroxyproline 1 g
- Polysorbate 40 commercialisé sous la dénomination de "Montanox 40 DF®" par la Société Seppic 0,75 g
- Polyéthylène oxyde à 8 moles d'oxyde d'éthylène (PEG-8) 1 g
- Triéthanolamine 0,02 g
- Eau déminéralisée q.s.p. 61,58 g

### Solution B

- Huile d'amande douce 5 g
- Huile de silicone volatile 5 g
- Parfum 0,3 g

### Solution C

- Hyaluronate de sodium 0,05 g
- Eau déminéralisée 10 g

Après homogénéisation de la dispersion A et des solutions B et C, on ajoute enfin la solution D suivante :

### Solution D

- Mélange d'acides polycarboxyvinyliques commercialisé sous la dénomination de "Carbopol 980®" par la Société Goodrich 0,3 g
- Conservateurs 0,3 g
- Triéthanolamine q.s.p. pH 6,5
- Eau déminéralisée 10,5 g

### EXEMPLE 9 : Fluide de jour

### Dispersion A

- Phytantriol 0,27 g
- N-2-hexyldécyloxycarbonyl-N-méthyl-glucamine telle qu'obtenue à l'exemple A 2,43 g
- Lécithine commercialisée sous la dénomination de "Epikuron 145 V®" par la Société Lucas Meyer 0,3 g
- Guanosine 0,01 g
- Glycérine 3 g
- Polyglycéryl-3 hydroxylauryléther commercialisé sous la dénomination de "Chimexane NF®" par la Société Chimex 0,5 g
- Eau déminéralisée 69,09 g

### Solution B

- Huile d'amande d'abricot 5 g
- Filtre solaire 1 g
- Huile de silicone volatile 5 g
- Parfum 0,3 g

### Solution C

- Mélange d'acides polycarboxyvinyliques commercialisé sous la dénomination de "Carbopol 980®" par la Société Goodrich 0,2 g
- Conservateurs 0,3 g
- Triéthanolamine q.s.p. pH 6,5
- Eau déminéralisée 12,6 g

### EXEMPLE 10 : Crème de jour

Selon le mode opératoire décrit à l'exemple 6, on prépare une crème de jour sous forme d'une dispersion par mélange des parties suivantes :

### Dispersion A

- Phytantriol 0,3 g
- Mélange de monoglycérides d'acide gras insaturé commercialisé sous la dénomination de "Myverol 18-99®" par la Société Eastman-Kodak 2,55 g
- Lécithine commercialisée sous la dénomination de "Epikuron 200®" par la Société Lucas Meyer 0,15 g
- Glycérine 3 g
- L-hydroxyproline 1 g
- D-Panthénol 0,5 g
- Polyphosphonate commercialisé sous la dénomination de "Dequest 2046®" par la Société Monsanto 0,1 g
- Lauroylglutamate de monosodium commercialisé sous la dénomination de "Acylglutamate LS-11®" par la Société Ajinomoto 0,1 g
- Eau déminéralisée 56,85 g

### Solution B

- Huile de jojoba 10 g
- Di-t-butyl hydroxybenzylidène camphre commercialisé sous la dénomination de "Mexoryl SAD®" par la Société Chimex 0,05 g
- Huile de silicone volatile 10 g
- Parfum 0,3 g

### Solution C

- Mélange d'acides polycarboxyvinyliques commercialisé sous la dénomination de "Carbopol 980®" par la Société Goodrich 0,4 g
- Conservateurs 0,3 g
- Lysine q.s.p. pH 6,5
- Eau déminéralisée 14,4 g

## Revendications

1. Composition sous forme d'une dispersion, caractérisée par le fait que ladite composition comprend :
(a) de 60 à 98 % en poids d'une phase aqueuse, et
(b) de 2 à 40 *%* en poids d'une phase huileuse,
ladite phase huileuse étant dispersée dans la phase aqueuse et stabilisée à l'aide de particules de gel cubique, lesdites particules étant essentiellement formées par :
(i) 0,1 à 15 % en poids par rapport au poids total de la composition, d'au moins un composé choisi parmi le 3,7,11,15-tétraméthyl-1,2,3-hexadecanetriol ou phytantriol, les dérivés N-2-alcoxycarbonyles de N-méthylglucamine, et les monoglycérides d'acide gras insaturé, et
(ii) 0,05 à 3 % en poids par rapport au poids total de la composition, d'un agent dispersant et stabilisant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

2. Composition selon la revendication 1, caractérisée par le fait que la proportion pondérale relative en composé (i) par rapport au poids de la phase huileuse est comprise entre 0,02/1 et 1/1.

3. Composition selon la revendication 2, caractérisée par le fait que ladite proportion pondérale est comprise entre 0,05/1 et 0,5/1.

4. Composition selon l'une quelconque des revendications précédentes, caractérisée par le fait que la proportion pondérale relative en composé (i) par rapport au poids dudit agent dispersant et stabilisant est comprise entre 2 et 200.

5. Composition selon l'une quelconque des revendications précédentes, caractérisée par le fait que ledit dérivé N-2-alcoxycarbonyle de N-méthylglucamine répond à la formule (I) suivante : 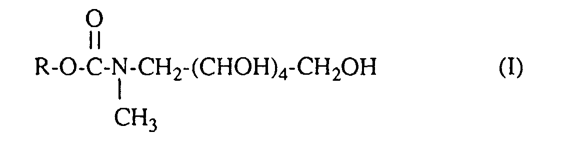 dans laquelle :
R représente un radical alkyle ramifié en C₆-C₁₈

6. Composition selon la revendication 5, caractérisée par le fait que ledit dérivé
N-2-alcoxycarbonyle de N-méthyl-glucamine est choisie parmi la N-2-hexyldécyloxycarbonyl-N-méthyl-glucamine,
la N-2-éthylhexyloxycarbonyl-N-méthyl-glucamine et la N-2-butyloctyloxycarbonyl-N-méthyl-glucamine.

7. Composition selon l'une quelconque des revendications 1 à 6, caractérisée par le fait que lesdites particules de gel cubique contiennent comme composé (i), un mélange constitué de 1 à 40 % en poids de phytantriol par rapport au poids du mélange et de 60 à 99 % en poids d'au moins un dérivé N-2-alcoxycarbonyle de N-méthyl-glucamine par rapport au poids du mélange.

8. Composition selon la revendication 7, caractérisée par le fait que lesdites particules de gel cubique contiennent un mélange constitué de 10 à 30 % en poids de phytantriol par rapport au poids du mélange et de 70 à 90 % en poids d'au moins un dérivé N-2-alcoxycarbonyle de N-méthyl-glucamine par rapport au poids du mélange.

9. Composition selon l'une quelconque des revendications 1 à 4, caractérisée par le fait que ledit monoglycéride d'acide gras insaturé est choisi parmi le monooléate de glycéryle et le monolinoléate de glycéryle.

10. Composition selon l'une quelconque des revendications 1 à 4 et 9, caractérisée par le fait que lesdites particules de gel cubique contiennent comme composé (i) un mélange constitué de 1 à 50 % en poids de phytantriol par rapport au poids du mélange, et de 50 à 99 % en poids d'au moins un monoglycéride d'acide gras insaturé par rapport au poids du mélange.

11. Composition selon la revendication 10, caractérisée par le fait que ledit mélange est constitué de 10 à 30 % in en poids de phytantriol par rapport au poids du mélange et de 70 à 90 % en poids d'au moins un monoglycéride d'acide gras insaturé par rapport au poids du mélange.

12. Composition selon l'une quelconque des revendications précédentes, caractérisée par le fait que ledit agent dispersant et stabilisant est choisi parmi :
(1) les alkyl ou alcényl éthers ou esters de polyol,
(2) les amino-acides N-acylés et leurs dérivés et les peptides N-acylés par un radical alkyle ou alcényle, et leurs sels,
(3) les alkyl ou alcényl éthers ou esters sulfates, leurs dérivés et leurs sels,
(4) les alkyl ou alcényl éthers ou esters gras polyoxyéthylénés,
(5) les acides alkyl ou alcényl carboxyliques polyoxyéthylénés et leurs sels,
(6) les N-alkyl ou alcényl bétaïnes,
(7) les alkyl ou alcényl triméthylammonium et leurs sels, et
(8) leurs mélanges.

13. Composition selon l'une quelconque des revendications précédentes, caractérisée par le fait que lesdites particules de gel cubique contiennent en outre de 0,0005 % à 5 % en poids d'au moins un lipide amphiphile ionique non hydrosoluble par rapport au poids total de la composition.

14. Composition selon la revendication 13, caractérisée par le fait que la proportion en lipide amphiphile ionique non hydrosoluble est de 0,001 % à 2 % en poids par rapport au poids total de la composition.

15. Composition selon l'une quelconque des revendications 13 et 14, caractérisée par le fait que ledit lipide amphiphile ionique non hydrosoluble est choisi parmi :
(i) les phospholipides,
(ii) les esters phosphoniques d'acide gras,
(iii) les dérivés N-acylés de l'acide glutamique non hydrosolubles,
(iv) le cétylsulfate de sodium,
(v) le cocoyl monoglycéride sulfate de sodium, et
(vi) les dérivés d'ammonium quaternaire non hydrosolubles.

16. Composition selon l'une quelconque des revendications précédentes, caractérisée par le fait que lesdites particules de gel cubique ont une taille moyenne d'environ 0,05 µm à 1µm.

17. Composition selon l'une quelconque des revendications précédentes, caractérisée par le fait que lesdites particules contiennent au moins un principe actif hydrophile et/ou lipophile.

18. Composition selon l'une quelconque des revendications précédentes, caractérisée par le fait que ladite phase huileuse se présente sous forme de gouttelettes de taille comprise entre 0,1 µm et 10 µm.

19. Composition selon l'une quelconque des revendications précédentes, caractérisée par le fait que ladite phase huileuse comprend au moins un principe actif lipophile.

20. Composition selon l'une quelconque des revendications précédentes, caractérisée par le fait que ladite phase aqueuse comprend au moins un principe actif hydrophile.

21. Composition selon l'une quelconque des revendications précédentes, caractérisée par le fait qu'elle est destinée à un usage cosmétique, dermatologique ou pharmaceutique.

22. Composition selon l'une quelconque des revendications précédentes, caractérisée par le fait qu'elle se présente sous forme d'un lait, d'une crème ou d'un sérum.

23. Procédé de préparation d'une composition sous forme d'une dispersion selon l'une quelconque des revendications précédentes, caractérisé par le fait qu'il consiste en une première étape à préparer une dispersion aqueuse de particules de gel cubique par fragmentation à l'aide d'un homogénéiseur d'un gel cubique formé par au moins un composé (i) tel que défini à la revendication 1, d'eau et d'au moins un agent dispersant et stabilisant (ii) tel que défini à la revendication 1 et dans une deuxième étape à ajouter à ladite dispersion obtenue une phase huileuse et à soumettre le mélange à une agitation mécanique.

24. Procédé selon la revendication 23, caractérisé par le fait que la première étape est réalisée en présence d'au moins un lipide amphiphile ionique non hydrosoluble et/ou d'au moins un pricinpe actif hydrophile et/ou lipophile.

25. Procédé selon l'une quelconque des revendications 23 et 24, caractérisé par le fait que la phase huileuse contient au moins un additif et/ou principe actif lipophile.

## Patentansprüche

1. Zubereitung in Form einer Dispersion, dadurch gekennzeichnet, dass die Zubereitung umfasst:
a) 60 bis 98 Gew.-% einer wässrigen Phase und
b) 2 bis 40 Gew.-% einer ölhaltigen Phase,
in der die ölhaltige Phase in der wässrigen Phase dispergiert ist und mit Hilfe von Partikeln eines kubischen Gels stabilisiert wird, wobei die Partikel im wesentlichen gebildet werden aus:
(i) 0,1 bis 15 Gew.-%, bezogen auf das Gesamtgewicht der Zubereitung, von mindestens einer Verbindung, ausgewählt unter 3,7,11,15-Tetramethyl-1,2,3-hexadecantriol oder Phytantriol, N-2-Alkoxycarbonyl-Derivaten von N-Methylglucamin und Monoglyceriden einer ungesättigten Fettsäure, und
(ii) 0,05 bis 3 Gew.-%, bezogen auf das Gesamtgewicht der Zubereitung, eines Dispergier- und Stabilisierungsmittels, wobei das Mittel unter den grenzflächenaktiven, bei Raumtemperatur wasserlöslichen Mitteln, die eine gesättigte oder ungesättigte, lineare oder verzweigte Kette einer Fettsäure mit 8 bis 22 Kohlenstoffatomen aufweisen, ausgewählt wird.

2. Zubereitung nach Anspruch 1, dadurch gekennzeichnet, dass das relative Gewichtsverhältnis der Verbindung (i), bezogen auf das Gewicht der ölhaltigen Phase, zwischen 0,02/1 und 1/1 liegt.

3. Zubereitung nach Anspruch 2, dadurch gekennzeichnet, dass das Gewichtsverhältnis zwischen 0,05/1 und 0,5/1 liegt.

4. Zubereitung nach einem der vorstehenden Ansprüche, dadurch gekennzeichnet, dass das relative Gewichtsverhältnis der Verbindung (i), bezogen auf das Gewicht des Dispergier- und Stabilisierungsmittel, zwischen 2 und 200 liegt.

5. Zubereitung nach einem der vorstehenden Ansprüche, dadurch gekennzeichnet, dass das N-2-Alkoxycarbonyl-Derivat von N-Methylglucamin der folgenden Formel (I) entspricht: 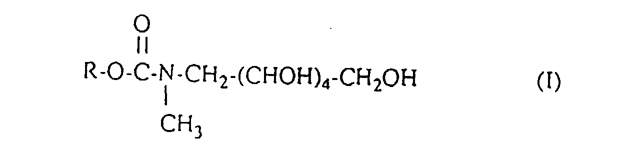 in der
R ein verzweigter C₆-C₁₈-Alkylrest ist.

6. Zubereitung nach Anspruch 5, dadurch gekennzeichnet, dass das N-2-Alkoxycarbonyl-Derivat von N-Methylglucamin unter N-2-Hexyldecyloxycarbonyl-N-methylglucamin, N-2-Ethylhexyloxycarbonyl-N-methylglucamin und N-2-Butyloctyloxycarbonyl-N-methylglucamin ausgewählt wird.

7. Zubereitung nach einem der vorstehenden Ansprüche 1 bis 6, dadurch gekennzeichnet, dass die Partikel des kubischen Gels als Verbindung (i) ein Gemisch enthalten, das zu 1 bis 40 Gew.-%, bezogen auf das Gewicht des Gemisches, aus Phytantriol und zu 60 bis 99 Gew.-%, bezogen auf das Gewicht des Gemisches, aus mindestens einem N-2-Alkoxycarbonyl-Derivat von N-Methylglucamin besteht.

8. Zubereitung nach Anspruch 7, dadurch gekennzeichnet, dass die Partikel des kubischen Gels ein Gemisch enthalten, das zu 10 bis 30 Gew.-%, bezogen auf das Gewicht des Gemisches, aus Phytantriol und zu 70 bis 90 Gew.-%, bezogen auf das Gewicht des Gemisches, aus mindestens einem N-2-Alkoxycarbonyl-Derivat von N-Methylglucamin besteht.

9. Zubereitung nach einem der Ansprüche 1 bis 4, dadurch gekennzeichnet, dass das ungesättigte Fettsäuremonoglycerid unter Glycerylmonooleat und Glycerylmonolinoleat ausgewählt wird.

10. Zubereitung nach einem der Ansprüche 1 bis 4 und 9, dadurch gekennzeichnet, dass die Partikel des kubischen Gels als Verbindung (i) ein Gemisch enthalten, das zu 1 bis 50 Gew.-%, bezogen auf das Gewicht des Gemisches, aus Phytantriol und zu 50 bis 99 Gew.-%, bezogen auf das Gewicht des Gemisches, aus mindestens einem ungesättigten Fettsäuremonoglycerid besteht.

11. Zubereitung nach Anspruch 10, dadurch gekennzeichnet, dass das Gemisch zu 10 bis 30 Gew.-%, bezogen auf das Gewicht des Gemisches, aus Phytantriol und zu 70 bis 90 Gew.-%, bezogen auf das Gewicht des Gemisches, aus mindestens einem ungesättigten Fettsäuremonoglycerid besteht.

12. Zubereitung nach einem der vorstehenden Ansprüche, dadurch gekennzeichnet, dass das Dispergier- und Stabilisierungsmittel ausgewählt wird unter:
(1) Alkyl- oder Alkenylethern oder -estern eines Polyols,
(2) N-acylierten Aminosäuren und deren Derivaten und durch einen Alkyl- oder Alkenylrest N-acylierten Peptiden und deren Salzen,
(3) Alkyl- oder Alkenylethern oder -estern von Sulfaten, deren Derivaten und deren Salzen,
(4) Alkyl- oder Alkenylethern oder -estern von polyethoxylierten Fettsäuren,
(5) polyethoxylierten Alkyl- oder Alkenylcarbonsäuren und deren Salzen,
(6) N-Alkyl- oder -Alkenylbetainen,
(7) Alkyl- oder Alkenyltrimethylammonium und Salzen davon und
(8) deren Gemischen.

13. Zubereitung nach einem der vorstehenden Ansprüche, dadurch gekennzeichnet, dass die Partikel des kubischen Gels außerdem 0,0005 Gew.-% bis 5 Gew.-%, bezogen auf das Gesamtgewicht der Zubereitung, von mindestens einem ionischen, wasserunlöslichen, amphiphilen Lipid enthalten.

14. Zubereitung nach Anspruch 13, dadurch gekennzeichnet, dass der Anteil des ionischen, wasserunlöslichen, amphiphilen Lipids 0,001 Gew.-% bis 2 Gew.-%, bezogen auf das Gesamtgewicht der Zubereitung, beträgt.

15. Zubereitung nach einem der Ansprüche 13 und 14, dadurch gekennzeichnet, dass das ionische, wasserunlösliche, amphiphile Lipid ausgewählt wird unter:
(i) Phospholipiden,
(ii) Phosphonsäureestern einer Fettsäure,
(iii) wasserunlöslichen, N-acylierten Derivaten der Glutaminsäure,
(iv) Natriumcetylsulfat,
(v) Natriumcocoylmonoglyceridsulfat und
(vi) wasserunlöslichen quaternären Ammoniumderivaten.

16. Zubereitung nach einem der vorstehenden Ansprüche, dadurch gekennzeichnet, dass die Partikel des kubischen Gels eine durchschnittliche Größe von ungefähr 0,05 µm bis 1 µm aufweisen.

17. Zubereitung nach einem der vorstehenden Ansprüche, dadurch gekennzeichnet, dass die Partikel mindestens einen hydrophilen und/oder lipophilen Wirkstoff enthalten.

18. Zubereitung nach einem der vorstehenden Ansprüche, dadurch gekennzeichnet, das die ölhaltige Phase in Form von Tröpfchen einer Größe zwischen 0,1 µm und 10 µm vorliegt.

19. Zubereitung nach einem der vorstehenden Ansprüche, dadurch gekennzeichnet, das die ölhaltige Phase mindestens einen lipophilien Wirkstoff umfasst.

20. Zubereitung nach einem der vorstehenden Ansprüche, dadurch gekennzeichnet, dass die wässrige Phase mindestens einen hydrophilen Wirkstoff umfasst.

21. Zubereitung nach einem der vorstehenden Ansprüche, dadurch gekennzeichnet, dass diese für eine kosmetische, dermatologische oder pharmazeutische Verwendung bestimmt ist.

22. Zubereitung nach einem der vorstehenden Ansprüche, dadurch gekennzeichnet, dass diese in Form einer Milch, einer Creme oder eines Serums vorliegt.

23. Verfahren zur Herstellung einer Zubereitung in Form einer Dispersion nach einem der vorstehenden Ansprüche, dadurch gekennzeichnet, dass dieses aus einem ersten Schritt der Herstellung einer wässrigen Dispersion von Partikeln eines kubischen Gels durch Fragmentierung mit Hilfe eines Homogenisators eines kubischen Gels, welches aus mindestens einer Verbindung (i) gemäß Anspruch 1, aus Wasser und aus mindestens einem Dispergier- und Stabilisierungsmittel (ii) gemäß Anspruch 1 gebildet wird, und einem zweiten Schritt des Zugebens erhaltenen Dispersion zu einer ölhaltigen Phase und des mechanischen Rührens des Gemisches besteht.

24. Verfahren nach Anspruch 23, dadurch gekennzeichnet, dass der erste Schritt in Anwesenheit von mindestens einem ionischen, wasserunlöslichen, amphiphilen Lipid und/oder von mindestens einem hydrophilen und/oder lipophilen Wirkstoff durchgeführt wird.

25. Verfahren nach einem der vorstehenden Ansprüche 23 und 24, dadurch gekennzeichnet, dass die ölhaltige Phase mindestens ein lipophiles Additiv und/oder einen lipophilen Wirkstoff enthält.

## Claims

1. Composition in the form of a dispersion, characterized in that the said composition comprises:
(a) from 60 to 98 % by weight of an aqueous phase, and
(b) from 2 to 40 % by weight of an oily phase, the said oily phase being dispersed in the aqueous phase and stabilized using cubic gel particles, the said particles being essentially formed of:
(i) 0.1 to 15 % by weight, relative to the total weight of the composition, of at least one compound chosen from 3,7,11,15-tetramethyl-1,2,3-hexadecanetriol or phytanetriol, N-2-alkoxycarbonyl derivatives of N-methylglucamine and unsaturated fatty acid monoglycerides, and
(ii) 0.05 to 3 % by weight, relative to the total weight of the composition, of a dispersing and stabilizing agent, the said agent being chosen from surface-active agents which are water-soluble at room temperature, containing a linear or branched, saturated or unsaturated fatty chain having from 8 to 22 carbon atoms.

2. Composition according to Claim 1, characterized in that the relative weight proportion of compound (i) relative to the weight of the oily phase is between 0.02/1 and 1/1.

3. Composition according to Claim 2, characterized in that the said weight proportion is between 0.05/1 and 0.5/1.

4. Composition according to any one of the preceding claims, characterized in that the relative weight proportion of compound (i) relative to the weight of the said dispersing and stabilizing agent is between 2 and 200.

5. Composition according to any one of the preceding claims, characterized in that the said N-2-alkoxycarbonyl derivative of N-methylglucamine corresponds to the following formula (I): 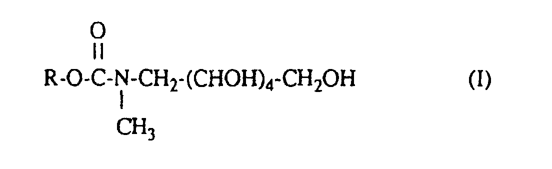 in which:
R represents a branched C₆-C₁₈ alkyl radical.

6. Composition according to Claim 5, characterized in that the said N-2-alkoxycarbonyl derivative of N-methylglucamine is chosen from N-2-hexyldecyloxycarbonyl-N-methylglucamine, N-2-ethylhexyloxycarbonyl-N-methylglucamine and N-2-butyloctyloxycarbonyl-N-methylglucamine.

7. Composition according to any one of Claims 1 to 6, characterized in that the said cubic gel particles contain, as compound (i), a mixture consisting-of from 1 to 40 % by weight of phytanetriol relative to the weight of the mixture and from 60 to 99 % by weight of at least one N-2-alkoxycarbonyl derivative of N-methylglucamine relative to the weight of the mixture.

8. Composition according to Claim 7, characterized in that the said cubic gel particles contain a mixture consisting of from 10 to 30 % by weight of phytanetriol relative to the weight of the mixture and from 70 to 90 % by weight of at least one N-2-alkoxycarbonyl derivative of N-methylglucamine relative to the weight of the mixture.

9. Composition according to any one of Claims 1 to 4, characterized in that the said unsaturated fatty acid monoglyceride is chosen from glyceryl monooleate and glyceryl monolinoleate.

10. Composition according to any one of Claims 1 to 4 and 9, characterized in that the said cubic gel particles contain, as compound (i), a mixture consisting of from 1 to 50 % by weight of phytanetriol relative to the weight of the mixture and from 50 to 99 % by weight of at least one unsaturated fatty acid monoglyceride relative to the weight of the mixture.

11. Composition according to Claim 10, characterized in that the said mixture consists of from 10 to 30 % by weight of phytanetriol relative to the weight of the mixture and from 70 to 90 % by weight of at least one unsaturated fatty acid monoglyceride relative to the weight of the mixture.

12. Composition according to any one of the preceding claims, characterized in that the said dispersing and stabilizing agent is chosen from:
(1) polyol alkyl or alkenyl ethers or esters,
(2) N-acylated amino acids and derivatives thereof and peptides N-acylated with an alkyl or alkenyl radical, and salts thereof,
(3) alkyl or alkenyl ether or ester sulphates, and the derivatives and salts thereof,
(4) polyoxyethylenated alkyl or alkenyl fatty ethers or esters,
(5) polyoxyethylenated alkyl or alkenyl carboxylic acids and salts thereof,
(6) N-alkyl or N-alkenyl betaines,
(7) alkyltrimethylammonium or alkenyltrimethylammonium and salts thereof, and
(8) mixtures thereof.

13. Composition according to any one of the preceding claims, characterized in that the said cubic gel particles also contain from 0.0005 % to 5 % by weight of at least one water-insoluble ionic amphiphilic lipid relative to the total weight of the composition.

14. Composition according to Claim 13, characterized in that the proportion of water-insoluble ionic amphiphilic lipid is from 0.001 % to 2 % by weight relative to the total weight of the composition.

15. Composition according to either of Claims 13 and 14, characterized in that the said water-insoluble ionic amphiphilic lipid is chosen from:
(i) phospholipids,
(ii) fatty acid phosphonic esters,
(iii) water-insoluble N-acylated derivatives of glutamic acid,
(iv) sodium cetyl sulphate,
(v) sodium cocoyl monoglyceride sulphate, and
(vi) water-insoluble quaternary ammonium derivatives.

16. Composition according to any one of the preceding claims, characterized in that the said cubic gel particles have a mean size approximately from 0.05 µm to 1 µm.

17. Composition according to any one of the preceding claims, characterized in that the said particles contain at least one hydrophilic and/or lipophilic active principle.

18. Composition according to any one of the preceding claims, characterized in that the said oily phase is in the form of droplets between 0.1 µm and 10 µm in size.

19. Composition according to any one of the preceding claims, characterized in that the said oily phase comprises at least one lipophilic active principle.

20. Composition according to any one of the preceding claims, characterized in that the said aqueous phase comprises at least one hydrophilic active principle.

21. Composition according to any one of the preceding claims, characterized in that it is intended for cosmetic, dermatological or pharmaceutical use.

22. Composition according to any one of the preceding claims, characterized in that it is in the form of a milk, a cream or a serum.

23. Process for the preparation of a composition in the form of a dispersion according to any one of the preceding claims, characterized in that it consists, in a first step, in preparing an aqueous dispersion of cubic gel particles by fragmentation, using a homogenizer, of a cubic gel formed of at least one compound (i) as defined in Claim 1, water and at least one dispersing and stabilizing agent (ii) as defined in Claim 1, and, in a second step, in adding an oily phase to the said dispersion obtained and in subjecting the mixture to mechanical stirring.

24. Process according to Claim 23, characterized in that the first step is performed in the presence of at least one water-insoluble ionic amphiphilic lipid and/or of at least one hydrophilic and/or lipophilic active principle.

25. Process according to either of Claims 23 and 24, characterized in that the oily phase contains at least one lipophilic additive and/or active principle.
